(19) Europäisches Patentamt
European Patent Office
Office européen des brevets

(11) Veröffentlichungsnummer: **0 089 586**
**B1**

(12) **EUROPÄISCHE PATENTSCHRIFT**

(45) Veröffentlichungstag der Patentschrift: **02.10.85**

(51) Int. Cl.⁴: **C 07 C 69/16,** C 07 C 67/29

(21) Anmeldenummer: **83102452.6**

(22) Anmeldetag: **12.03.83**

(54) **2-Alkyl-2,4-diacyloxy-3-butenale.**

(30) Priorität: **24.03.82 DE 3210707**

(43) Veröffentlichungstag der Anmeldung: **28.09.83 Patentblatt 83/39**

(45) Bekanntmachung des Hinweises auf die Patenterteilung: **02.10.85 Patentblatt 85/40**

(84) Benannte Vertragsstaaten: **BE CH DE FR GB IT LI NL**

(56) Entgegenhaltungen:
**EP - A - 0 005 432**
**EP - A - 0 068 372**

(73) Patentinhaber: **BASF Aktiengesellschaft, Carl-Bosch-Strasse 38, D-6700 Ludwigshafen (DE)**

(72) Erfinder: **Weitz, Hans-Martin, Dr., Auf dem Koeppel 40, D-6702 Bad Duerkheim (DE)**
Erfinder: **Fischer, Rolf, Dr., Bergstrasse 98, D-6900 Heidelberg (DE)**
Erfinder: **Paust, Joachim, Dr., Ringstrasse 3, D-6708 Neuhofen (DE)**

**EP 0 089 586 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue 2-Alkyl-2,4-diacyloxy-3-butenale.

Es ist aus der EP-PS 5 452 bekannt, daß bei der Umsetzung von 1-Acyloxy-2-alkyl-1,3-butadienen mit Carbonsäuren und Sauerstoff in Gegenwart von Palladium oder Platin enthaltenden Katalysatoren 2-Alkyl-1,1,4-triacyloxy-2-butene entstehen.

Es wurden nun neue 2-Alkyl-2,4-diacyloxy-3-butenale der Formel

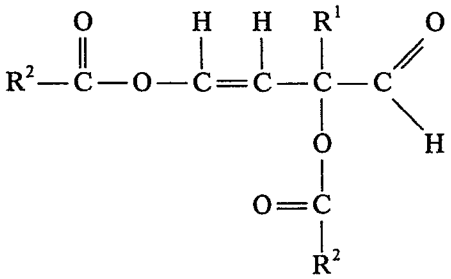

(I)

gefunden, in der $R^1$ einen Alkylrest mit 1 bis 5 Kohlenstoffatomen und $R^2$ eine Wasserstoffatom oder einen Alkylrest mit 1 bis 15 Kohlenstoffatomen, einen cycloaliphatischen Rest mit 5 bis 7 Kohlenstoffatomen oder einen aromatischen Rest bedeutet.

Alkylreste mit 1 bis 5 bzw. 1 bis 15 C-Atomen sind z. B. Methyl-, Ethyl-, n-Propyl-, i-Propyl-, n-Butyl-, i-Butyl-, tert.-Butyl-, n-Pentyl-, Palmityl- oder Stearylreste. Als cycloaliphatische Reste kommen z. B. Cyclopentyl-, Cyclohexyl- oder Cycloheptylreste in Betracht. Aromatische Reste sind z. B. Phenylreste, die gegebenenfalls durch Alkyl- oder Halogenreste substituiert sind, wie Phenyl-, 2-Chlorphenyl-, 2,4-Dichlorphenyl-, 2-Methylphenyl- oder 2,4-Dimethylphenylreste.

Von besonderem technischem Interesse sind 2-Alkyl-2,4-diacyloxy-3-butenale der Formel

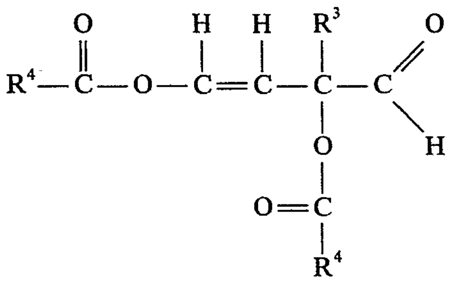

(II)

in der $R^3$ einen Alkylrest mit 1 bis 3 Kohlenstoffatomen und $R^4$ ein Wasserstoffatom oder einen Alkylrest mit 1 bis 3 Kohlenstoffatomen bedeutet. Besonders bevorzugt ist 2-Methyl-2,4-diacetoxy-3-butenal.

Die neuen 2-Alkyl-2,4-diacyloxy-3-butenale der Formel I werden z. B. hergestellt, indem man 1,3-Butadiene der Formel

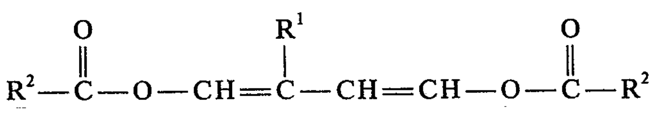

(III)

in der $R^1$ und $R^2$ die obengenannte Bedeutung haben, in unter den Reaktionsbedingungen inerten Lösungsmitteln mit Sauerstoff abgebenden Verbindungen behandelt.

Ein Verfahren, bei dem man 1,3-Butadiene der Formel III in Gegenwart von Carbonsäuren mit Sauerstoff abgebenden Verbindungen behandelt, wird in der EP-A-68 372 beschrieben. Nach den Angaben dieser älteren Patentanmeldung setzt man die Butadienverbindungen z. B. in Essigsäure mit einer Sauerstoff abgebenden Verbindung um, wobei 2-Alkyl-4,4-diacyloxy-2-butenale erhalten werden.

Die Reaktion zur Herstellung der 2-Alkyl-2,4-diacyloxy-3-butenale läßt sich für den Fall der Umsetzung von 2-Methyl-1,4-diacetoxy-1,3-butadien mit m-Chlorperbenzoesäure in Chloroform zu 2-Methyl-2,4-diacetoxy-3-butenal durch die folgenden Formeln veranschaulichen:

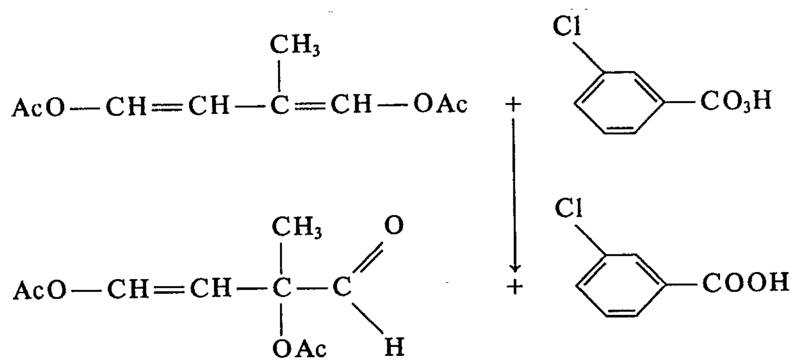

Als 2-Alkyl-1,4-diacyloxy-1,3-butadiene der Formel III seien beispielsweise die folgenden Verbindungen genannt: 2-Methyl-, 2-Ethyl-, 2-n-Propyl-, 2-Butyl-, 2-Pentyl-1,4-diacetoxy-1,3-butadien, 2-Methyl-1-acetoxy-4-palmityloxy-1,3-butadien, 2-Methyl-1-cyclohexyloxy-4-acetoxy-1,3-butadien und 2-Methyl-1-benzoyloxy-4-acetoxy-1,3-butadien.

Ausgangsstoffe der Formel III lassen sich z. B. durch Acetylierung von 2-Alkyl-4-acyloxy-2-butenalen mit Acetanhydrid (J. org. Chem. 41, 2625 [1976]) oder durch Pyrolyse von 2-Alkyl-3,4-diacetoxy-tricyclo[4,2,1,$0^{2,5}$]-non-7-enen (Journal of Chemical Society, Chemical Communications 1974, 956) herstellen.

Als Sauerstoff abgebende Verbindungen sind z. B. aromatische Percarbonsäuren, wie Perbenzoesäure, m-Chlorperbenzoesäure oder organische Hydroperoxide, wie tert.-Butylhydroperoxid oder Cumolhydroperoxid geeignet. Verbindungen dieser Art sind z. B. in Ullmanns Encyclopädie der Technischen Chemie, 4. Auflage, Band 10, Seiten 563 bis 567, genannt. Die Sauerstoff abgebende Verbindung kann auch in Gegenwart von Katalysatoren, z. B. solchen, die Metalle, wie Bor, Zinn, Arsen, Titan, Zirkon, Vanadin, Molybdän, Wolfram enthalten oder aus entsprechenden Verbindungen bestehen, angewendet werden. Pro Mol 1,3-Dien der Formel III wendet man z. B. 0,5 bis 10 Mol, insbesondere 1 bis 1,5 Mol, der Sauerstoff abgebenden Verbindung an.

Man arbeitet in Gegenwart von unter den Reaktionsbedingungen inerten Lösungsmitteln. Als Lösungsmittel dieser Art kommen z. B. Carbonsäureester, wie Essigsäuremethylester, chlorierte Kohlenwasserstoffe, wie Methylenchlorid, Chloroform, Tetrachlorkohlenstoff oder 1,2-Dichlorethan, Kohlenwasserstoffe, wie Alkane, Benzol und alkylierte Benzole, Ether, wie Diethylether, Tetrahydrofuran oder Dioxan in Betracht. Pro Mol Ausgangsverbindung der Formel III verwendet man zweckmäßigerweise 0,1 bis 80 Mol, insbesondere 2 bis 60 Mol, des unter den Reaktionsbedingungen inerten Lösungsmittels.

Das Verfahren wird zweckmäßig bei Temperaturen zwischen —20 und 200° C, insbesondere bei 20 bis 120° C, durchgeführt. Man kann chargenweise oder kontinuierlich, drucklos oder unter Druck arbeiten. Nicht umgesetzte 1,3-Diene können gegebenenfalls nach der Umsetzung destillativ von den entstandenen 2-Alkyl-2,4-diacyloxy-3-butenalen abgetrennt und erneut für die erfindungsgemäße Umsetzung verwendet werden.

Die Umsetzung kann man bei diskontinuierlicher Arbeitsweise z. B. wie folgt vornehmen: Einer Lösung des 1,3-Diens in dem jeweiligen Lösungsmittel wird bei der Reaktionstemperatur und dem Reaktionsdruck die Sauerstoff abgebende Verbindung zugeführt. Nach beendeter Zugabe wird gegebenenfalls noch nachgerührt. Durch das auf Raumtemperatur abgekühlte Reaktionsgemisch wird gegebenenfalls Stickstoff geleitet. Anschließend wird nach dem Abdestillieren des Lösungsmittels fraktioniert destilliert. Dabei werden gegebenenfalls nicht umgesetzte Ausgangsverbindungen von den gewünschten Aldehyden abgetrennt.

Die nach dem Verfahren zugänglichen neuen 2-Alkyl-2,4-diacyloxy-3-butenale der Formel I lassen sich z. B. durch Behandlung mit aliphatischen Carbonsäuren in Abwesenheit von starken Säuren zu 2-Alkyl-4,4-diacyloxy-2-butenalen umlagern und stellen daher wertvolle Zwischenprodukte für die Herstellung von Terpenen wie Retinal, $\beta$-Carotin (DE-OS 2 357 810) und Apocarotinoiden dar.

## Beispiel

Zu einer Lösung von 92 g 2-Methyl-1,4-diacetoxy-1,3-butadien in 1300 g Chloroform wurden bei 30 ± 2° C unter Rühren innerhalb von zwei Stunden 1300 g einer Chloroform-Lösung gegeben, die 86 g m-Chlorperbenzoesäure und 15 g m-Chlorbenzoesäure enthielt. Man rührte eine weitere Stunde bei dieser Temperatur nach. Unumgesetzte Persäure war nicht mehr im Reaktionsgemisch nachweisbar. Die Chloroformphase wurde zuerst mit insgesamt 1000 $cm^3$ Wasser, das 84 g Natriumbicarbonat enthielt, dann mit 200 $cm^3$ Wasser gewaschen und über Magnesiumsulfat getrocknet. Das Chloroform wurde am Rotationsverdampfer abgezogen. Durch fraktionierte Destillation des Rückstands wurden 50,5 g eines Gemisches vom Siedepunkt 80 bis 96° C/0,5 mbar, $n_D^{20}$ = 1,4598 erhalten, das nach dem $^1$H-NMR-Spektrum 31 g 2-Methyl-2,4-diacetoxy-3-butenal (31%, bezogen auf eingesetztes Dien) und 16,8 g 2-Methyl-4,4-diacetoxy-2-butenal (17%, bezogen auf eingesetztes Dien) enthielt.

$^1H$-NMR-Spektren (Lösungsmittel $CDCl_3$, TMS als innerer Standard)

(E)-2-Methyl-2,4-diacetoxy-3-buten:

$\delta$ = 1,55 (s, $-CH_3$, 3 H), ca. 2,15 (s, $-CO-CH_3$, 6 H); 5,48 (d, $-C-CH=$, J = 13 Hz, 1 H), 7,41 (d, $-O-CH=$, J = 13 Hz, 1 H), 9,28 (s, $-CHO$, 1 H).

(Z)-2-Methyl-2,4-diacetoxy-3-buten:

$\delta$ = 1,58 (s, $-CH_3$, 3 H), ca. 2,15 (s, $-CO-CH_3$, 6 H), 5,15 (d, $-C-CH=$, J = 7 Hz, 1 H), 7,10 (d, $-O-CH=$, J = 7 Hz, 1 H), 9,37 (s, $-CHO$, 1 H).

**Patentansprüche**

1. 2-Alkyl-2,4-diacyloxy-3-butenale der Formel

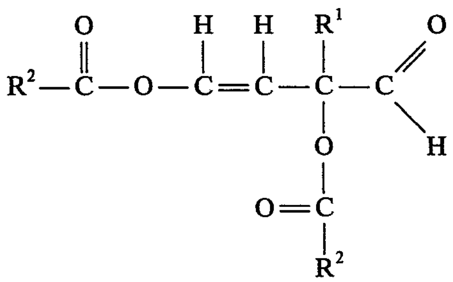

(I)

in der $R^1$ einen Alkylrest mit 1 bis 5 Kohlenstoffatomen und $R^2$ ein Wasserstoffatom oder einen Alkylrest mit 1 bis 15 Kohlenstoffatomen, einen cycloaliphatischen Rest mit 5 bis 7 Kohlenstoffatomen oder einen aromatischen Rest bedeutet.

2. 2-Alkyl-2,4-diacyloxy-3-butenale der Formel

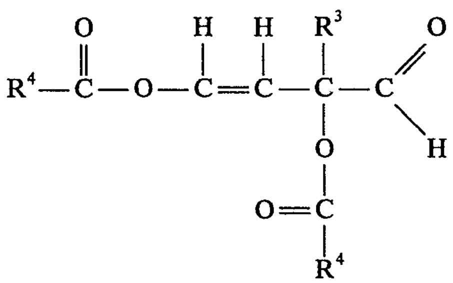

(II)

in der $R^3$ einen Alkylrest mit 1 bis 3 Kohlenstoffatomen und $R^4$ ein Wasserstoffatom oder einen Alkylrest mit 1 bis 3 Kohlenstoffatomen bedeutet.

3. 2-Methyl-2,4-diacetoxy-3-butenal.

**Claims**

1. A 2-alkyl-2,4-diacyloxy-but-3-enal of the formula

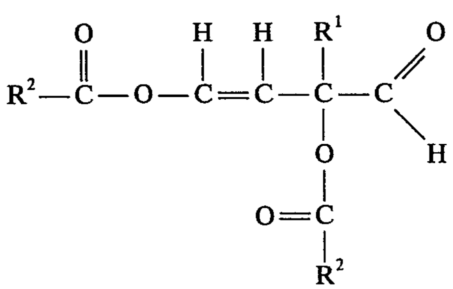

(I)

where $R^1$ is alkyl of 1 to 5 carbon atoms and $R^2$ is hydrogen, alkyl of 1 to 15 carbon atoms, a cycloaliphatic radical of 5 to 7 carbon atoms or an aromatic radical.

2. A 2-alkyl-2,4-diacyloxy-but-3-enal of the formula

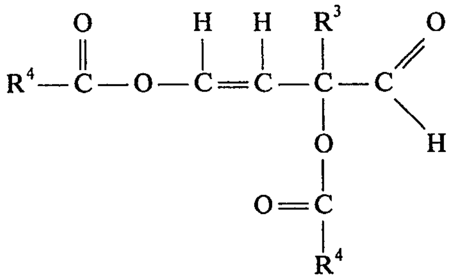

(II)

where $R^3$ is alkyl of 1 to 3 carbon atoms and $R^4$ is hydrogen or alkyl of 1 to 3 carbon atoms.

3. 2-Methyl-2,4-diacetoxy-but-3-enal.

**Revendications**

1. 2-alkyl-2,4-diacyloxy-3-buténals de formule

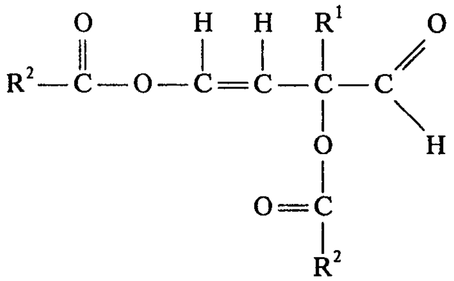

(I)

dans laquelle $R^1$ représente un radical alkyle à 1—5 atomes de carbone et $R^2$ un atome d'hydrogène ou un radical alkyle à 1—15 atomes de carbone, un radical cycloaliphatique à 5—7 atomes de carbone ou un radical aromatique.

2. 2-alkyl-2,4-diacyloxy-3-buténals de formule

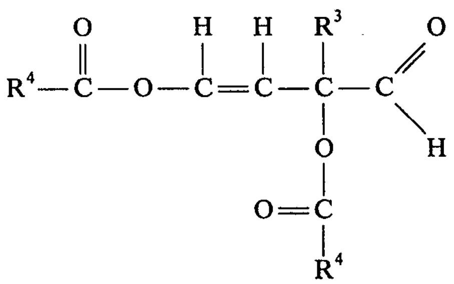

(II)

dans laquelle $R^3$ représente un radical alkyle à 1—3 atomes de carbone et $R^4$ un atome d'hydrogène ou un radical alkyle à 1—3 atomes de carbone.

3. 2-méthyl-2,4-diacétoxy-3-buténal.